# EUROPÄISCHE PATENTANMELDUNG

(11) **EP 1 374 862 A2**
(43) Veröffentlichungstag der Anmeldung: **02.01.2004**
(21) Anmeldenummer: 03013295.5
(22) Anmeldetag: 13.06.2003
(51) Int. Cl.: A61K 31/136, A61K 31/517, A61K 31/522, A61K 31/00, A61K 31/663, A61K 38/00, A61K 39/395, A61K 31/7088, G01N 33/50, C12N 15/11, A61P 43/00, A61P 9/10

(54) **Verwendung einer auf den Phosphorylierungsgrad von BAD einwirkenden Substanz zur Behandlung von Atherosklerose**

(30) Priorität: 13.06.2002 DE 10226420; 05.07.2002 US 189188
(71) Anmelder: Eucro European Contract Research GmbH & Co. KG, 41460 Neuss (DE)
(72) Erfinder: Klumpp, Susanne, Prof, Dr., 481149 Münster (DE); Krieglstein, Josef, Prof, Dr., Dr., 35091 Cölbe (DE)
(74) Vertreter: König, Gregor Sebastian, Dipl.-Biol.

(57) **Zusammenfassung**

Die Erfindung betrifft die Verwendung einer Substanz, die gezielt auf den Phosphorylierungsgrad von BAD einwirkt, zur Verhinderung bzw. Behandlung der Atherosklerose. Femer werden mit der Erfindung geeignete Screeningverfahren zur Verfügung gestellt, die das Auffinden geeigneter Substanzen ermöglichen.

## Beschreibung

Kardiovaskuläre Erkrankungen in Form von Herzinfarkt, Schlaganfall und peripheren Gefäßverschlüssen zählen in den Industrienationen zu den Haupttodesursachen. Diesen Erkrankungen liegt häufig eine Atherosklerose zugrunde. Als Atherosklerose wird der morphologische Symptomenkomplex von Veränderungen der inneren Gefäßwand bestehend aus lokalen Ansammlungen von Fetten, komplexen Kohlenhydraten, Blutbestandteilen sowie fibrösem Gewebe und Kalzium verbunden mit Veränderungen der mittleren Gefäßwand verstanden. Dies führt insgesamt zu einer Verdickung und Verhärtung der Arterienwand.

Die Entwicklung atherosklerotischer Schädigungen (Läsionen) umfaßt im wesentlichen drei Prozesse, nämlich die Migration, Proliferation und Akkumulation von Makrophagen, T-Lymphozyten und glatten Muskelzellen in der Gefäßinnenwand, die Bildung von extrazellulärer Matrix durch die glatten Muskelzellen sowie die Akkumulation von Lipiden in Makrophagen, Muskelzellen und der extrazellulären Matrix (Ross, R. (1993). The pathogenesis of atherosclerosis: a perspective for the 1990s. Nature 1993 April 29; 362 (6423) 801-809).

Die Entstehung atherosklerotischer Läsionen wird durch eine Verletzung oder Dysfunktion des Endothels ausgelöst. Diese initiiert u. a. eine Thrombozytenadhäsion und -aggregation und damit eine Sezemierung von Wachstumsfaktoren wie beispielsweise des PDGF (Platelet derived growth factor). Darauf folgt eine Proliferation intimaler glatter Muskelzellen und letztlich - durch die Bildung einer neuen Gefäßinnenwand (Neointima) - eine Gefäßwandverdickung. Femer verursachen Endothelläsionen eine Permeabilitätssteigerung der Gefäßwand, die neben einer erhöhten Expression von Adhäsionsmolekülen eine Einwanderung von Makrophagen und Leukozyten bewirkt, was nachfolgend zu einer inflammatorischen Reaktion führen kann.

Neben Rauchen, Hypertonie und der Diabetes mellitus stellt ein erhöhter Lipidblutspiegel, insbesondere ein erhöhter Cholesterolblutspiegel von mehr als 200 mg/dl, einen bedeutenden Risikofaktor für die Entstehung der Atherosklerose dar. Als Plasmalipide kommen Neutralfette (Triglyceride), Phospholipide, Cholesterol (Cholesterin), Cholesterolester und freie Fettsäuren vor. Diese liegen im Blut nicht in freier Form, sondern als sog. Lipoproteine, d. h. gebunden an Trägerproteine, vor. Diese Trägerproteine unterteilen sich aufgrund ihres Verhaltens bei der Ultrazentrifugation bzw. der Elektrophorese in mehrere Typen, nämlich in Chylomikronen, Very-low-density-Lipoproteine (VLDL), Intermediate-density-Lipoproteine (IDL), Low-densitiy - Lipoproteine (LDL) und High-density-Lipoproteine (HDL), und werden als Apolipoproteine bezeichnet. Der Hauptbestandteil der LDL ist das Cholesterin.

Hohe LDL-Konzentrationen führen zu einer vermehrten Ablagerung von Cholesterin in den Gefäßwandzellen, indem LDL Cholesterin zu den periphären Zellen transportiert. Damit begünstigen sie Entstehung der Atherosklerose. Erhöhte HDL-Werte haben dagegen eine protektive Wirkung.

Daneben wird den LDL eine besonders atherogene Wirkung zugeschrieben, da sie durch reaktive Sauerstoffspezies oder durch aus dem umgebenden Gewebe freigesetzten Enzyme zu oxLDL oxidiert werden können. Infiltrierende Makrophagen können die oxLDL durch ihre Scavenger-Rezeptoren aufnehmen und damit aufgrund der intrazellulären Lipidansammlung zu Schaumzellen werden, die pro-inflammatorische, prothrombische und atherogene Substanzen sezernieren. Dabei kommt es auch zur Freisetzung von Zytokinen und damit zu einer Proliferation der vaskulären glatten Muskelzellen (de Winter M.P. et al., Macrophage scavenger receptor class A: A multifunctional receptor in atherosclerosis. Arterioscler Thromb Vasc Biol. 2000 Feb; 20 (2): 290-297.

Die zunächst kleinen Areale des LDL akkumulieren mit zunehmender Progression der Atherosklerose zu einem Plaque. Durch Kalziumanlagerungen können daraus fortgeschrittene Plaques entstehen, die das Gefäß massiv einengen. Wenn es zu einer Plaqueruptur kommt, kann diese eine Thrombusbildung mit nachfolgendem Gefäßverschluß verursachen, was wiederum eine Ischämie des umliegenden Gewebes und ggf. einen Infarkt zur Folge haben kann.

Da ein erhöhter Lipidspiegel als Hauptrisikofaktor für die Atherosklerose gilt, setzen die bekannten Therapiekonzepte für ihre Behandlung und Prävention an der Senkung des erhöhten Plasmalipidspiegel an, wobei im wesentlichen die Reduktion der LDL und ihrer Vorläufer VLDL im Vordergrund steht. Dazu ist es beispielsweise bekannt, Fibrato- oder Nikotinsäurederivate einzusetzen, die durch eine Verminderung der Synthese und einen verstärkten Abbau potentiell atherogener VLDL die Konzentration von Plasmatriglyceriden und Plasmacholesterin senken. Auch lonenaustauscherharzen können zur Reduzierung der Konzentration der potentiell atherogenen LDL eingesetzt werden.

In einem anderen therapeutischen Ansatz zur Behandlung der Atherosklerose wird durch die Applikation von Inhibitoren der HMG-CoA-Reduktase als Schlüsselenzym der Cholesterinbiosynthese und eine Steigerung der Überexpression der LDL-Rezeptoren in der Leber versucht, eine Senkung der LDL-Konzentration zu erreichen. Diese auch als Statine bezeichneten HMG-CoA-Reduktase-inhibitoren können eine Reduktion des potentiell atherogenen LDL-Cholesterins bis zu 60 % bewirken.

Des weiteren ist bekannt, daß apoptotische Prozesse an der Entstehung der Atherosklerose beteiligt sind. Als Apoptose (oder programmierter Zelltod) wird das Sterben von Zellen als Reaktion auf Streßfaktoren oder toxische Substanzen sowie eine gezielte Selbstmordreaktion bezeichnet. Die Apoptose erfolgt durch eine gerichtete Aktivierung von Signaltransduktionswegen, die zu einer charakteristischen DNA-Fragmentierung, einer Ruptur der Nukleusmembran, einer Chromatinkondensation, dem Schrumpfen der Zellen und Membranausstülpungen führen (Kerr J. F. et al (1972). Apoptosis: a basic biological phenomenon with wide-ranging implications in tissue kinetics. Br J Cancer 26, 1972 Aug; 26 (4): 239-257; Searle J. et al. (1982) Necrosis and apoptosis: distinct models of cell death with fundamentally different significance. Pathol Annu 17, 229-259). Die Eliminierung der toten Zellen erfolgt anschließend im Wege der Phagozytose durch die Nachbarzellen.

Für die Apoptoseinduktion sind verschiedene Signaltransduktionswege beschrieben. In Säugetieren wird die Apoptose hauptsächlich durch die Caspasen, Apaf 1 (apoptotic protease-activating factor) und die Bcl2 Familie reguliert (siehe Adams JM et al (1998). The Bcl-2 protein family: arbiters of cell survival. Science 1998 Aug. 28; 281 (5381): 1322-1326; Ashkenazi et al (1998). Death receptors: signalling and modulation. Science 1998 Aug. 28; 281 (5381): 1305-1308).

Verschiedene Untersuchungen an humanen Atheroektomien weisen Apoptose in atherosklerotischen Plaques nach (Han et al (1995). Evidence for apoptosis in human atherogenesis and in a rat vascular injury model. Am J Pathol 1995 Aug.;147 (2), 267-277; Hegyi, L. et al (1996). Foam cell apoptosis and the development of the lipid core of human atherosclerosis. J. Pathol 1996 Dec; 180 (4), 423-429). So wurden apoptopische Prozesse in Makrophagen und glatten Muskelzellen gezeigt. Ferner ist bekannt, daß nach einer Plaqueruptur Apoptose hauptsächlich in der fibrösen Kappe, an der Rupturstelle, nahe von Fetteinlagerungen und um den nekrotischen Kem herum erfolgt (Crisby et al (1997). Cell death in human atherosclerotic plaques involves both oncosis and apoptosis. Artherosclerosis 1997 Apr.; 130 (1-2), 17-27).

Vieles deutet darauf hin, daß die Zerstörung des Endothels durch Apoptose ein initiales Ereignis bei der Atheroskleroseentwicklung darstellt, da pro-atherosklerotische Faktoren wie oxLDL, Angiotensin II oder reaktive Sauerstoffspezies in vitro ebenso die Apoptose in Endothelzellen induzieren (Dimmler et al (1997). Angiotensin II induces apoptosis of human endothelian cells. Protective effects of nitiric oxide. Circ Res. 81, 970-976; Dimmler et al (1997). Suppression of apoptosis by nitric oxide via inhibition of ICE-like and CPP32-like proteases. J. Exp. Med. 185, 601-608; Hermann et al (1997). Shear stress inhibits H₂O₂ induced apoptosis of human endothelial cells by modulation of the glutathione redox cycle and nitric oxide synthase. Arterioscler Thromb Vasc Biol 1997 Dec.; 17 (12) 3588-3592). Die Apoptose der Endothelzellen zerstört dabei die Integrität der Endothelzellschicht und trägt dadurch zu den pro-inflammatorischen Reaktionen bei, welche zur Ausbildung der Plaques führen. Die Apoptose der Endothelzellen ist für den Prozeß der Plaque-Erosion und Thrombozytenaggregation daher entscheidend.

Ferner wird die Rolle von NO als wichtiger physiologischer Mediator auf die Apoptose-Signaltransduktion diskutiert. Frühere Studien zeigten, daß NO die Apoptose in verschiedenen Zelltypen induzieren kann. Neuere Untersuchungen deuten dagegen auf anti-apoptotische Effekte von NO und den Schutz von Endothelzellen hin (Dimmler et al (1997). Angiotensin II induces apoptosis of human endothelian cells. Protective effects of nitiric oxide. Circ Res. 81, 970-976; Dimmler et al (1997). Suppression of apoptosis by nitric oxide via inhibition of ICE-like and CPP32-like proteases. J. Exp. Med. 185, 601-608; Hermann et al (1997). Shear stress inhibits H₂O₂ induced apoptosis of human endothelian cell by modulation of the glutathione redox cycle and nitric oxide synthetase. Arterioscler Thromb Vasc Biol 17, 3588-3592). Danach schützen sich Endothelzellen vor Apoptose, indem sie u. a. auch Stickstoffmonoxid freisetzen, das die den programmierten Zelltod katalysierenden Enzyme hemmt. Sauerstoffradikale inaktivieren jedoch Stickstoffmonoxid, und erhalten damit die Möglichkeit des Zelltods. So sterben selbst bei Anwesenheit von NO Endothelzellen ab. Die toten Zellen werden zwar durch neue Zellen ersetzt, letztere zeigen jedoch eine niedrigere biologische Aktivität im Hinblick auf die Freisetzung von Stickstoffmonoxid. Durch die reduzierte Freisetzung von Stickstoffmonoxid wird wiederum das Fortschreiten der Atherosklerose begünstigt. Die verminderte Stickstoffmonoxidproduktion hat femer eine Vermehrung der glatten Gefäßmuskelzellen zur Folge, so daß die Blutgefäße weiter verengt werden - mit der bekannten Folge der Sauerstoffunterversorgung. Auch dieser Mechanismus weist auf die grundlegende Bedeutung der Apoptose von Endothelzellen hinsichtlich der Entwicklung der Atherosklerose hin.

Die Verhinderung der Apoptose der Endothelzellen steht daher im Vordergrund der Bestrebungen, einen wirksamen Präventions- und Therapieansatz für die Atherosklerose aufzufinden. Seit längerem wird versucht, die erkannten Zusammenhänge zwischen der Apoptose der Endothelzellen und der Atherosklerose - insbesondere die Kenntnisse über die die Atherosklerose initiierenden Faktoren - zur Entwicklung eines Wirkstoffs zu nutzen. Bislang blieben diese Bestrebungen jedoch erfolglos, obwohl Erkenntnisse über die Beteiligung des BAD (Bcl-2/Bcl-X_{L}-Antagonist causing cell death) an der Regulation der Apoptose vorliegen.

BAD gehört zu einer als Bcl-2-Familie bezeichneten Gruppe Apoptose regulierender Proteine, die neben pro-apoptotische Proteine wie Bax und anti-apoptotische Proteine wie Bcl-2 und Bcl-X umfaßt. BAD dimerisiert selektiv mit Bcl-2 und Bcl-X (Yang E., et al. (1995) BAD, a heterodimeric partner for Bcl-X_{L} and Bcl-2 displaces Bax and promotes cell death. Cell 1995 Jan. 27; Vol. 80 (2) 285-291) und verdrängt damit Bax als Bindungspartner von Bcl-X_{L,} Die anti-apoptotischen Effekte von Bcl-2/Bcl-X_{L} werden durch diese Interaktion verhindert. Daneben induziert das nunmehr freie Bax apoptotische Reaktionen (Korsmeyer SJ. (1999). BCL-2 gene family and the regulation of programmed cell death. Cancer Res 1999 Apr. 1; 1:59 (7 suppl) 1693-1700). Vermutlich handelt es bei dieser Reaktion um den entscheidenden Mechanismus, durch welchen BAD die Apoptose fördert.

BAD kann in verschiedenen phosphorylierten Formen vorliegen. So ist es bereits seit längerem bekannt, daß sowohl Serin¹¹² als auch Serin ¹³⁶'phosphoryliert sein kann. Dies wurde bereits in vivo nachgewiesen. Ebenso ist bekannt, daß die Dephosphorylierung dieser Reste durch PP1 (Proteinphosphatase 1) und PP2B (Proteinphosphatase Typ 2 B) katalysiert wird (Ayllon V. et al. (2000), Proteinphosphatase 1 α is a ras-acitvated BAD phospatase that regulates interleukin-2 deprivation-induced apoptosis. EMBO J. 2000 May 15; Vol. 19 (10) 2237-2246; Wang HG et al., (1999) Ca²⁺ induced apoptosis through calcineurin dephosphorylation of BAD. Science 1999 Apr. 9; Vol. 284 (5412) 339-343). Daneben wurde erst jüngst nachgewiesen, das BAD auch am Serin¹⁵⁵ phosphoryliert sein kann. Die Phosphorylierung dieses Serins erfolgt in vitro durch die Proteinkinase A (PKA). Diese wird durch cAMP aktiviert.

Liegt BAD phosphoryliert vor (nachfolgend Phospho-BAD), wird die Bindung an Bcl-2/Bcl-X unmöglich und Phospho-BAD dissoziiert. Phospho-BAD kann dagegen an das sog 14-3-3-Protein binden. Das nunmehr freie Bcl-X_{L} kann seine anti-apoptotische Wirkung entfalten und mit Bax interagieren (Zha J. et al., (1996) Serin phosphorylation of death agonist BAD in response to survival factor results in binding to 14-3-3 not Bcl-X_{L}, Cell 1996 Nov. 15; Vol. 87 (4) 619-628). Damit begünstigt die Phosphorylierung des BAD anti-apoptotische Prozesse (Lizcano J. M., Morice N. and Cohen P. (2000) Regulation of BAD by cAMP dependent protein kinase is mediated via phosphorylation of a novel side, Ser¹⁵⁵. Biochem, J. 2000 Jul. 15; Vol.: 349 (Pt 2): 547-557; Tan Y. et al (2000) BAD Ser¹⁵⁵ Phosphorylation regulates BAD/BclX_{L} interaction and cell survival. J. Biol. Chem. 2000 Aug. 18; 275 (33): 25865 - 25869).

Dieser Mechanismus liegt wohl dem bekannten Phänomen zugrunde, daß HDL anti-atherogen wirkt. So wurde gezeigt, daß HDL Endothelzellen vor Apoptose schützen können, indem sie Proteinkinase Akt induzieren. Die Proteinkinase Akt Induktion ist ebenso durch die aus den HDL-Partikeln isolierten Lysophospholipide möglich (Nofer et al (2001) Suppression of endothelial cell apoptosis by high density lipoproteins (HDL) and HDL-associated lysosphingolipids. J. Biol Chem 2001 Sep. 14; 276 (37): 34480-5).

Die bekannten Therapien der Atherosklerose greifen übereinstimmend unspezifisch in den Lipidblutspiegel ein, insbesondere durch den Lipidblutspiegel senkende Substanzen. Dies gilt auch für Ansätze, mit denen eine Verhinderung der Apoptose in Endothelzellen einhergeht (beispielsweise durch eine HDL-Erhöhung). Auch sie setzen primär an der Regulation des Lipidblutspiegels an.

Davon ausgehend liegt der vorliegenden Erfindung die Aufgabe zugrunde, ein Verfahren sowie einen Stoff oder eine Stoffgruppe bereitzustellen, mit dem sich in die die Atherosklerose begünstigende Apoptose der Endothelzellen mittelbar oder unmittelbar eingreifen läßt.

Der Erfindung liegt der Gedanke zugrunde, gezielt anti-apoptotische Effekte zur Regulation der die Atherosklerose in nachteiliger Weise begünstigenden Apoptose dadurch zu fördem und zu nutzen, daß das BAD überwiegend in seiner anti-apoptotische Phospho-Form gehalten bzw. darin überführt wird. Dies kann dem Grunde nach über eine unmittelbare oder mittelbare Verhinderung der Dephosphorylierung des Phospho-BAD bzw. eine unmittelbare oder mittelbare Stimulation oder Induktion der Phosphorylierung erfolgen.

Gemäß einer ersten Ausführungsvariante der vorliegenden Erfindung werden zur Verhinderung oder Reduktion der Dephosphorylierung erfindungsgemäß vorzugsweise Wirkstoffe eingesetzt, die Serin-Threonin-Phosphatasen, insbesondere die Proteinphosphatase Typ 2 C hemmen (PP2C).

Als Wirkstoff wird in diesem Zusammenhang jeder biologisch oder pharmazeutisch aktiver Stoff angesehen, der direkt oder indirekt zu einer Verminderung oder Reduktion der Expression dieser Phosphatasen oder ihrer biologischen Aktivität führen kann. Diese Definition schließt demnach ebenso Oligonukleotid-Wirkstoffe wie z.B. Antisense-Oliogonukleotide ein.

Die Proteinphosphatase Typ 2C (PP2C) gehört zu den eukaryontischen Serin-Threonin-Phosphatasen (PP1, PP2A, PP2B, PP2C), deren Dephosphorylierungsaktivität von Mn²⁺ oder Mg²⁺ Ionen abhängig ist (McGowan, CH. and Cohen P. (1988) Protein phosphatase-2C from rabbit skeletal muscle and liver: an Mg²⁺-dependent enzyme. Meth. Enzymol. 1988; Vol. 159, 416-426). PP2C ist an der Regulation der Apoptose beteiligt. Die Überexpression dieses Enzyms ist letal (Wenk J. und Mieskes G. (1995) Cytosolic and nuclear localization of protein phophatase -2C beta 1 in COS and BHK cells. Eur. J Cell Biol 1995 Dec; 68 (4), 377-386); Cheng A. et al (1999) Dephosphorylation of cyclin-dependent kinases by type 2C protein phosphatases. Genes Dev. 1999 Nov. 15; 13 (22), 2946-2957). Über den genauen Mechanismus, über den PP2C in die apoptotische Regulation eingreift, liegen bislang keine gesicherten Erkenntnisse vor.

PP2C wurde bislang in verschiedenen Geweben, wie beispielsweise neuronalem Gewebe, nachgewiesen. In Endothelzellen konnte der Nachweis bisher aber nicht erbracht werden. Entsprechend war die Regulation der Apoptose in diesen Zellen über Phospho-BAD und PP2C unbekannt. Daher gab es bislang keine Bestrebungen, an der Regulation der Konzentration von BAD bzw. Phospho-BAD als Therapie-Ansatz zur Behandlung und Prophylaxe der Atherosklerose anzuknüpfen.

Es ist nun gelungen, in Endothelzellen neben BAD auch PP2C nachzuweisen und zu zeigen, daß Phospho-BAD ein Substrat für PP2C darstellt. Es ist daher vorteilhaft, einen insbesondere die PP2C (als Serin-Threonin-Phosphatase) hemmenden Wirkstoff einzusetzen und dadurch gezielt die Dephosphorylierung des Phospho-BAD zu verhindern. Damit wird ein wirkungsvoller Therapieansatz zur Behandlung und Prophylaxe der Atherosklerose bereitgestellt.

Von der PP2C ist bekannt, daß sie durch ungesättigte Fettsäuren stimuliert wird (Klumpp, S., Selke, D., Hermes Mayer J. (1998) Protein phosphatase 2C active at physiological Mg²⁺: stimulation by unsaturated fatty acids. FEBS letters 1998 Oct. 23; 437 (3): 229-232). Dieser Umstand ist vorliegend bedeutsam, da in Endothelzellen Fettsäuren in hohen Konzentrationen vorliegen durch die die Apoptose in Endothelzellen durch Induktion der PP2C eingeleitet werden kann, wodurch das Atheroskleroserisiko gesteigert wird. Fettsäuren können daher die Apoptose der Endothelzellen durch Stimulation der PP2C Aktivität induzieren. Es stellt daher einen besonderen Vorteil der Erfindung dar, bei der Therapie oder Prävention der Atherosklerose auch hemmend an der Dephosphorylierung von Phospho-BAD durch eine durch Fettsäuren aktivierte Phosphatase PP2C in Endothelzellen anzusetzen. Dies kann erfindungsgemäß dadurch erfolgen, indem die Fettsäureaktivierung gezielt verhindert wird.

Die Begünstigung der Phosphorylierung des BAD kann gemäß einer weiteren Ausführungsform der vorliegenden Erfindung über die Applikation eines Wirkstoffes erfolgen, der eine erhöhte Expression, Induktion, Aktivierung oder Stimulation von BAD phosphorylierenden Proteinkinasen bewirkt. Diese Kinasen sind vorzugsweise die Proteinkinase B (PKB bzw. Akt), das Mitogen-aktivierte Protein (MAP) Kinase-aktivierte Proteinkinase-1 (MAPKAP-K1 bzw. RSK) und die Proteinkinase A. Diese phosphorylieren das Serin-112, Ser-136 bzw. das Ser-155 des BAD. Die erfindungsgemäße Begünstigung der Phosphorylierung des BAD kann beispielsweise mittels eines Wirkstoffes erfolgen, der regulierend in die Signaltransduktions- oder Expressionsprozesse eingreift und dadurch eine Steigerung der Kinaseaktivität bewirkt oder durch einen direkten Aktivator der BAD phosphorylierenden Kinasen.
Mit der Erfindung wird somit ein wirksamer Ansatz zur therapeutischen Behandlung und Prophylaxe der Atherosklerose bereitgestellt, da über die vorhandene Menge des aktiven pro-apoptotischen BAD - auf die erfindungsgemäß über den Phosphorylierungszustand Einfluß genommen wird - insbesondere dem initiale Ereignis der Atheroskleroseentwicklung, nämlich der Apoptose der Endothelzellen, entgegengewirkt werden kann. Die erfindungsgemäßen Substanzen können daher in vorteilhafter Weise zur Herstellung eines Therapeutikums zur Behandlung bzw. Prävention der Atherosklerose verwendet werden.

In der bevorzugten Ausführungsform der Erfindung wird ein hohes Niveau an Phospho-BAD - und damit ein niedriges Niveau an BAD - erhalten, indem die Dephosphorylierung durch Phosphatasen, insbesondere durch PP2C, verhindert oder wenigstens reduziert wird. Dies kann auf verschiedene Weisen erfolgen, nämlich beispielsweise durch die Verwendung:
a) eines Phosphatase-Inhibitors,
b) eines Wirkstoffs, der an Phospho-BAD bindet, dieses stabilisiert und/oder ggf. für Phosphatasen unzugänglich macht,
c) eines Antisense-Oligonukleotids, das die Expression einer ausgesuchten Phosphatase, insbesondere der PP2C, in den Endothelzellen unterdrückt.

Es hat sich als besonders vorteilhaft herausgestellt, die erfindungsgemäße Verhinderung der Dephosphorylierung von Phospho-BAD mittels Antisense-Oligonukleotiden und insbesondere durch deren Verwendung zur Komplexierung der PP2C mRNA zur Inhibition der PP2C durchzuführen. Die Oligonukleotide können sowohl einen synthetischen als auch einen natürlichen Ursprung haben. So können sie beispielsweise aus Hefe gewonnen werden.

Mit der Erfindung werden zudem Verfahren zur Verfügung gestellt, die es ermöglichen, geeignete Inhibitoren von Phosphatasen oder Bindungspartner für Phospho-BAD aufzufinden, die erfindungsgemäß zur Herstellung eines geeigneten Therapeutikums eingesetzt werden können, um die Dephosphorylierung des BAD und somit die die Atherosklerose begünstigende Apoptose zu verhindern.

Gemäß einer Ausführungsform eines solchen Verfahrens kann ein Wirkstoff identifiziert und damit zur Verfügung gestellt werden, der PP2C - und damit die Dephosphorylierung von Phospho-BAD - inhibiert. Das Verfahren basiert auf der in vitro Inkubation von PP2C mit Phospho-BAD bei Anwesenheit einer Testsubstanz und der nachfolgenden Evaluierung, ob die Dephosphorylierung von Phospho-BAD vermindert oder gehemmt wurde.

Mittels eines solchen Identifizierungssystems können somit Substanzen aufgefunden werden, die im erfindungsgemäßen Ansatz zur Herstellung eines Therapeutikums zur Behandlung und Prävention der Atherosklerose eingesetzt werden können. Der besondere Vorteil liegt darin, daß die regulatorische Wirkung von Phospho-BAD auf die Apoptose mittelbar auch von der Aktivität der PP2C abhängig ist.

Gemäß einer vorteilhaften Weiterbildung des erfindungsgemäßen Verfahrens wird im Inkubationsschritt wenigstens eine Fettsäure hinzugegeben, welche PP2C aktivieren kann. Dadurch können gezielt Substanzen identifiziert werden, die selektiv die Aktivierung der PP2C durch Fettsäuren verhindern. So kann in vorteilhafter Weise ein Wirkstoff gefunden werden, der insbesondere die Apoptose von Endothelzellen bei einer atherogenen Fettsäurekonzentration verhindert, d.h. die aufgefundene Substanz wirkt selektiv dieser Wirkung des erhöhten Fettsäurespiegels entgegen. Diese hohe Spezifität ist von besonderem Vorteil, da anderenfalls unerwünschte Nebeneffekte durch die Beeinträchtigung weiterer Regulationsprozeße unter Beteiligung der PP2C auftreten können. Eine ungesteuerte Hemmung der Apoptose könnte z.B. zu einem erhöhten Krebsrisiko führen, da der programmierte Zelltod ein wichtiges Instrument der Zellregulation ist. Die selektive Inhibierung der Aktivierung der PP2C durch Fettsäuren reduziert in vorteilhafter Weise die zu erwartenden Nebenwirkungen der als Therapeutikum einsetzbaren Substanz, da speziell nur die durch atherogene Faktoren ausgelöste bzw. gesteigerte Apoptose vermindert bzw. verhindert wird.

Gemäß einer weiteren Ausführungsform wird ein Verfahren zur Identifizierung einer Substanz zur Regulierung der geschilderten apoptotischen Vorgänge vorgeschlagen, bei dem nach der Induktion der Apoptose in Endothelzellen ein enzymatisch aktiver Zellextrakt gewonnen, dieser anschließend mit Phospho-BAD versetzt und mit einer oder mehreren Testsubstanzen inkubiert wird. Nachfolgend erfolgt die Evaluierung auf eine mögliche Hemmung der Dephosphorylierung des Phospho-BAD durch die Testsubstanz.

Dieses Verfahren berücksichtigt in vorteilhafter Weise die physiologischen Besonderheiten der Endothelzellen und identifiziert damit Testsubstanzen mit besonderer Eignung für den erfindungsgemäßen Therapieansatz. Dieses Verfahren kann auch unter Verwendung einer Endothelzellkultur durchgeführt werden, wobei dann auf die vorherige Gewinnung eines Zellextraktes verzichtet werden kann, da der Test direkt an den Endothelzellen erfolgt. Die Überprüfung, inwieweit die Endothelzellen Apoptose unterlaufen, kann mittels der in Beispiel 2 aufgeführten Methoden erfolgen.

Die Induktion der Apoptose kann bevorzugt durch wenigstens eine PP2C aktivierende Fettsäure erfolgen. Dieses Vorgehen hat den Vorteil, daß damit - wie oben beschrieben - ein selektiver Wirkstoff bereitgestellt wird.

In einer weiteren Ausführungsform wird ein Verfahren zur Identifizierung einer Substanz zur Verfügung gestellt, das ein Screenen einer Substanzbibliothek mit PP2C als Target zur Identifizierung von Bindungspartnem umfaßt. Im Anschluß daran werden die derart isolierten Substanzen mit PP2C und Phospho-BAD inkubiert und auf ihre Wirkung auf die Dephosphorylierung des Phospho-BAD analysiert. Dies kann zunächst in einem zellfreien System erfolgen und dann nach Bestätigung der Wirkung an Endothelzellkulturen getestet werden.

Damit geht der Vorteil einher, daß nur solche Substanzen auf eine Modulation der PP2C-Aktivität getestet werden, von denen aufgrund des Screenings bekannt ist, daß sie an PP2C binden können. Dadurch wird in vorteilhafter Weise die Vielzahl möglicher Testsubstanzen erheblich eingeschränkt und die Effizienz des Verfahrens erhöht.

Als Substanzbibliotheken können erfindungsgemäß chemische, natürliche, biologische oder Peptid-Bibliotheken ausgewählt werden. Diverse derartiger Bibliotheken sind im Stand der Technik bekannt und in Verbindung mit der Erfindung einsetzbar. Zur Identifizierung von potentiellen Peptidwirkstoffen sind insbesondere Phagen oder Phagemidbibliotheken geeignet.

Weiterhin wird ein Verfahren bereitgestellt, bei dem eine Substanzbibliothek mit Phospho-BAD als Target zur Identifizierung von Bindungspartnem gescreent wird. Anstelle von Phospho-BAD können auch phosphorylierte Peptide als Targets verwendet werden, die vorzugsweise den entsprechenden Phosphorylierungsbereichen um Serin 112, 136 und 155 entsprechen. Dies hat den Vorteil, daß hier gezielt nur Bindungspartner ermittelt werden, die an die vorliegend relevanten phosphoryliebaren BAD-Stellen binden und nicht an andere Bereiche des Phospho-BAD. Mögliche Peptidtargets zur Identifizierung von potentiellen Wirksubstanzen sind:
112-Stelle: R(107) SRMSS(112)YPDRG(117)
136-Stelle: R(132)GSRS(136)APPNN(141)
155-Stelle: R(149)ELRRMS(155)DEFEGS(161)

Diese Sequenzen sind Antigensequenzen aus Maus. Anstelle dieser können femer die entsprechenden menschlichen BAD-Peptidsequenzen eingesetzt werden. Selbstverständlich kann der ausgewählte Peptidbereich auch in Richtung des N- oder C-Terminus vergrößert oder verschoben werden. Vorzugsweise weisen die eingesetzten Peptidfragmente eine Länge von wenigstens 8 Aminosäuren auf.

Auch hier kann anschließend eine Inkubation der identifizierten Liganden mit PP2C und Phospho-BAD sowie eine Evaluierung ihrer Wirkung auf die Dephosphorylierung des Phospho-BAD durchgeführt werden.

Diese Ausführungsform hat den Vorteil, daß gezielt Substanzen ermittelt werden, die an das phosphorylierte BAD binden und somit die Phosphorylierungsstelle für PP2C unzugänglich machen können. Auf diese Weise können geeignete Inhibitoren des Dephosphorylierungsschritts identifiziert werden, welche nicht direkt auf PP2C wirken. Dies hat den Vorteil, daß die Aktivität von PP2C und somit die Regulation anderer Stoffwechselwege, an denen PP2C regulativ beteiligt ist, nicht gestört wird. In diesem Zusammenhang ist das Screenen von Peptidbibliotheken (bspw. Phagemidbibliotheken) vorteilhaft, da Peptidwirkstoffkandidaten die für Proteine und Peptide typischen Wechselwirkungen ausbilden können und dadurch spezifisch binden. Femer kann die Spezifität der Bindung der Peptidwirkstoffkandidaten an Phospho-BAD gezielt durch mehrere Screeningschritte und Rekombination geeigneter Kandidaten erhöht werden.

Zudem wird ein Verfahren zur Herstellung eines Therapeutikums zur Behandlung der Atherosklerose zur Verfügung gestellt, wobei in einem ersten Schritt eine Substanz mit inhibierender Wirkung auf die hier relevanten apoptotischen Vorgänge in Endothelzellen vorzugsweise mittels eines erfindungsgemäßen Verfahren identifiziert wird. In einem weiteren Schritt wird diese Substanz in ausreichender Menge hergestellt und mit physiologisch verträglichen Trägersubstanzen versetzt. Dadurch wird eine Substanz zur Herstellung eines Therapeutikum bzw. eine pharmakologisch wirksame Substanz zur Verfügung gestellt, welche zur Behandlung der Atherosklerose eingesetzt werden kann.

Gemäß einer vorteilhaften Weiterbildung der oben genannten Verfahren wird PP2C aus der Gruppe von PP2Cα; PP2Cβ; PP2Cγ; PP2Cδ und deren Subtypen ausgewählt. Diese Subtypen sind in den nachfolgenden Publikationen beschrieben, auf welche zu Zwecken der Offenbarung vollumfänglich Bezug genommen wird (Terasava et al. (1993) Molecular cloning of a novel isotyp of Mg²⁺ -dependent protein phosphatase β enriched in brain and heart. Arch. Biochem. Biophys. 307, 342-349; Hou et al., (1994) Molecular cloning of cDNAs encoding to isoforms of protein phosphatase 2Cβ from mouse testis, Biochem. and Mol. Biol. Int. 32, 773-780; Kato et al., (1995) Molecular cloning and expression of mouse Mg dependent protein phosphatase typ 2Cβ-4. Arch. Biochem. 318, 387-393). Vorzugsweise wird als PP2C PP2Cα und/oder PP2Cβ, insbesondere PP2α, eingesetzt.

Erfindungsgemäß kann Phospho-BAD eingesetzt werden, welches in den Positionen Serin¹⁵⁵, Serin¹¹² und/oder Serin¹³⁶ phosphoryliert ist. Von all diesen Phosphorylierungsstellen ist es bekannt, daß sie die Interaktion von BAD mit Bcl-2 und Bcl-X_{L} verhindern. So fördert beispielsweise die Phosphorylierung des Serin¹³⁶ die Bindung von BAD an das 14-3-3 Protein. Besonders bevorzugt wird BAD eingesetzt, welches in der Position Serin¹⁵⁵ phosphoryliert ist. Die Phosphorylierung des Serin¹⁵⁵ verhindert die Bindung von BAD an Bcl-X_{L} und ist somit entscheidend für die regulatorische Funktion. Vorliegend konnte nachgewiesen werden, daß PP2C die Position P-Ser¹⁵⁵ im Vergleich zu P-Ser¹¹² und P-Ser¹³⁶ bevorzugt dephosphoryliert. Diese Selektivität zeigt die bedeutende Rolle von PP2C bei der Regulation der Apoptose. Aufgrund dieser Selektivität ist es insbesondere vorteilhaft, einen Inhibitor zu finden, welcher die Dephosphorylierung am Serin¹⁵⁵ effektiv verhindert, insbesondere auch aufgrund der besonderen Bedeutung des Ser¹⁵⁵ in der Verhinderung der Apoptose. Ferner kann BAD eingesetzt werden, welches am Ser¹⁷⁰ phosphoryliert ist.

Gemäß einer vorteilhaften Weiterbildung der oben genannten Verfahren wird das Phospho-BAD radioaktiv markiert, um den Nachweis zu erleichtern. Dazu kann ([³²P]) BAD eingesetzt werden. Als weitere Nachweismethode können erfindungsgemäß auch Antikörper gegen Phospho-BAD und/oder BAD (ohne Einfluß auf den Phosphorylierungsstatus) eingesetzt werden. Femer ist es vorteilhaft, selektiv Antikörper gegen P-Ser¹¹², P-Ser¹³⁶, P-Ser¹⁵⁵ einzusetzen. Dadurch kann bestimmt werden, ob die Testsubstanz gezielt die Dephosphorylierung an einer der genannten Phosphorylierungsposition verhindert.

Zur Aktivierung von PP2C werden vorzugsweise Fettsäuren mit einer Länge von wenigstens 15 C-Atomen ausgewählt, die mindestens einfach ungesättigt sind und wenigstens eine freie Carboxylgruppe aufweisen. Beispiele für derartige, die PP2C selektiv aktivierenden Fettsäuren sind Ölsäure, Arachidonsäure und Ginkolsäure. Diese führen nachgewiesenermaßen zu einer 10 bis 15-fachen Steigerung der PP2C Aktivität.

Dem Testansatz werden femer Mn²⁺ oder Mg²⁺ Ionen zugegeben, vorzugsweise Mg²⁺ in einer Konzentration von 0,5 bis 1,5 mM. Ferner enthält der Testansatz vorzugsweise noch weitere Zusätze, wie beispielsweise Puffer, weitere Kofaktoren und/oder ähnliches.

Die dargestellten Verfahren können in vorteilhafter Weise eingesetzt werden, um damit Substanzen aufzufinden, welche die Dephosphorylierung von Phospho-BAD durch eine PP2C verhindern und somit als Therapeutikum zur Behandlung der Atherosklerose eingesetzt werden können.

Gemäß einer Ausführungsform wird ein Inhibitor einer PP2C verwendet, um ein Therapeutikum herzustellen, daß die Apoptose in Endothelzellen und damit auch die Atherosklerose verhindern oder zumindest vermindern kann.

Besonders vorteilhaft ist es, wenn die erfindungsgemäß einsetzbare Substanz die Apoptose in Endothelzellen durch - vorzugsweise selektive - Hemmung der Aktivierung der PP2C durch Fettsäuren unterbindet.

Insbesondere vorteilhaft ist es, wenn die erfindungsgemäß einsetzbare Substanz kompetitiv die Bindung von PP2C aktivierenden Fettsäuren an PP2C verhindert. Diese Substanz kann beispielsweise selbst eine Fettsäure, eine modifizierte Fettsäure oder ein Fettsäureanalogon sein, welche die Bindungsstelle der die PP2C aktivierenden Fettsäuren blockiert, jedoch selber keine Aktivierung der PP2C bewirkt.

Inhibierende Derivate der die PP2C aktivierenden Fettsäuren können ferner beispielsweise Biphosphonat-Fettsäuren und/oder deren Derivate sein. Diese beinhalten ferner den Vorteil, daß sie mittels der Biphosphonatgruppe an die Kalkablagerungen in den Gefäßen binden und somit gezielter am gewünschten Wirkungsort wirken können. Diese können aus den jeweiligen Fettsäuren ausgehend von der Säuregruppe phosphoniert werden. Beispiele sind Biphosphonsäuren und/oder deren Salze, vorzugsweise gemäß der allgemeinen Formel 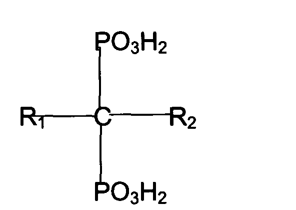 in welcher R1 ein linearer oder verzweigter Alkylrest mit 1 bis 12 Kohlenstoffatomen ist, der gegebenenfalls substituiert sein kann durch Substituenten wie Aminogruppen, N-Mono- bzw. Dialkylaminogruppen, wobei die Alkylgruppen 1 bis 12 C-Atome und/oder SH-Gruppen enthalten können, oder ein substituierter oder unsubstituierter carbo- oder heterocyclischer Arylrest, der ggf. ein oder mehrere Heteroatome und als Substituenten verzweigte und/oder unverzweigte Alkylreste mit 1 bis 12 C-Atomen, freie oder monorespektive dialkylierte Aminogruppen mit 1 bis 6 C-Atomen oder Halogenatome aufweisen kann und R₂ gleich OH, ein Halogenatom, vorzugsweise Cl, H oder NH₂ ist. Beispiele für derartige Substanzen sind Ibandronsäure, Etidronsäure, Clodronsäure, Pamidronsäure, Alendronsäure, Risedonsäure, Tiludronsäure, Zoledronsäure, Cimadronsäure, Neridronsäure, Olpadronsäure, 3-Pyrrol-1-hydroxypropan-1-1-diphosphonsäure und/oder Minodronsäure, Diphosphonat-1,1-Dicarbonsäure (DPD) und/oder 1-Methyl-1-hydroxy-1.1-diphosphonsäure (MDP).

Die erfindungsgemäßen Wirkstoffe können ferner zu diagnostischen Zwecken (beispielsweise zur Lokalisation von Plaques) oder als Kontrastmittel eingesetzt werden. Der diesbezügliche Einsatz von Biphosphonatfettsäurederivaten ist besonders vorteilhaft, da diese (wie erwähnt) mittels der Biphosphonatgruppe an Kalkablagerungen binden und somit Atheroskleroseherde (Plaques) aufspüren können. So können sie beispielsweise im Rahmen von szintigraphischen Methoden eingesetzt werden. Vorzugsweise werden dazu DPD und MDP eingesetzt. Ferner kann die Biphosphonatgruppe mittels radioaktivem Technetium-99-meta an der Biphosphonatgruppe markiert werden, Der Fettsäurerest kann dann bei Rezeptorbindung das radioaktive Signal an die Bindungsstelle Plaque transportieren, wodurch eine Diagnose möglich ist. Ferner können die Biphosphonatfettsäure-Derivate in Kombination mit sogenannten paramagnetischen Eisen-Nanopartikeln als Magnetresonanz-Tomographie (MRT)-Kontrastmittel eingesetzt werden. Dadurch können in vorteilhafter Weise Plaques aufgespürt und eine entstehende oder bereits vorhandene Atherosklerose bereits im Frühstadium diagnostiziert werden, bevor verheerende gesundheitliche Schäden auftreten.

Ebenso kann die erfindungsgemäß einzusetzende Substanz ein negativer Regulator einer durch Fettsäuren aktivierbaren PP2C sein. Eine solche Substanz kann beispielsweise an das entsprechende regulatorische Zentrum der PP2C binden und dadurch deren Aktivierung verhindern.

Gemäß einer weiteren Ausführungsform kann die inhibitorische Substanz an das aktive Zentrum der PP2C binden und somit kompetitiv die Bindung von Phospho-BAD durch PP2C unterbinden. Diesbezüglich ist es vorteilhaft, wenn die Substanz in ihrer dreidimensionalen Struktur im wesentlichen der dreidimensionalen Struktur des Phospho-BAD entspricht und ein nicht oder nur langsam umsetzbares Substratanalogon darstellt. Jedoch sind auch anders strukturierte kompetetive Hemmstoffe erfindungsgemäß einsetzbar.

Gemäß einer weiteren Ausführungsform können erfindungsgemäß Antikörper oder Antikörperfragmente gegen PP2C eingesetzt werden. Spezifisch gegen regulatorische Bereiche der PP2C gerichtete Antikörper oder Antikörperfragmente haben den Vorteil, daß Bindungsstellen für die PP2C aktivierende Faktoren blockiert werden können. Dadurch wird eine Aktivierung der PP2C verhindert.

Des weiteren kann erfindungsgemäß eine Substanz eingesetzt werden, welche an Phospho-BAD bindet und die Phosphatgruppe für PP2C unzugänglich macht. Eine solche Substanz kann beispielsweise ein Peptid oder Polypeptid sein. Der Einsatz eines Polypeptids bietet den Vorteil, das dieses aus einer Peptidbibliothek mittels etablierter Screeningverfahren isoliert werden kann und auch auf eine hohe Spezifität hin weiterentwickelt oder modifiziert werden kann. Ein solches Polypeptid sollte vorzugsweise einen Interaktionsbereich wie beispielsweise eine Bindungstasche aufweisen, mittels dem sie Phospho-BAD binden und somit die Phosphorylierungsstelle für PP2C unzugänglich machen kann. Sofern dieser Bereich eine Bindungstasche darstellt, kann diese in ihrer dreidimensionalen Struktur beispielsweise im wesentlichen der Antigenbindungsstelle von Antikörpern gegen Phospho-BAD entsprechen. Da derartige Antikörper bereits bekannt sind, kann eine solche Substanz auf einfache Weise aus den bekannten Aminosäuresequenzen der Antigenbindungsstellen generiert werden. Die Bindungstasche kann auch modifiziert werden, um eine besonders hohe Spezifität für Phospho-BAD in den jeweiligen Phosphorylierungsstellen zu erzielen und gegebenenfalls Nebenreaktionen mit anderen Zellbestandteilen zu verhindern.

Gemäß einer vorteilhaften Weiterbildung entspricht die Bindungstasche der erfindungsgemäß zur Herstellung eines Therapeutikums einzusetzenden Substanz in ihrer dreidimensionalen Struktur im wesentlichen der Antigenbindungsstelle eines Antikörpers gegen Phospho-BAD Serin¹¹², Phospho-BAD Serin¹³⁶, Phospho-BAD Serin¹⁵⁵. Dabei ist es besonders vorteilhaft, die Bindungstasche in ihrer dreidimensionalen Struktur der Antigenbindungsstelle gegen Phospho-BAD Serin¹⁵⁵ nachzubilden, da gezeigt wurde, daß PP2C vorzugsweise diese Stelle dephosphoryliert und diese bedeutsam für die regulatorische Funktion von BAD ist.

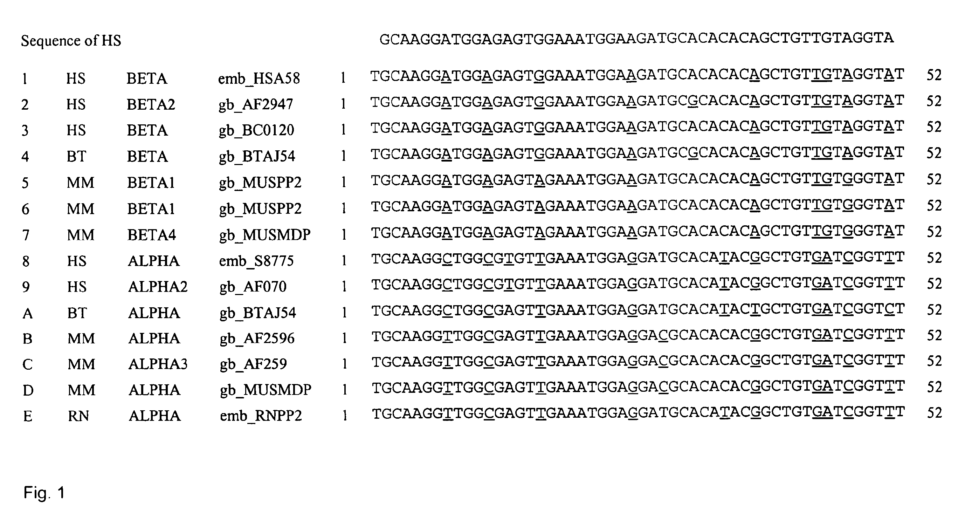

Die Dephosphorylierung von Phospho-BAD kann zudem auch durch die Hemmung der Bildung von PP2C in der Zelle verhindert werden. Dazu kann ein Molekül eingesetzt werden, welches die mRNA von PP2C komplexiert und dadurch ihre Translation verhindert. Bei dem Molekül kann es sich um ein zumindest teilweise einzelsträngiges Oligonukleotid handeln, das mit der mRNA der PP2C hybridisiert (sog. Antisenese-Oligonukleotid). Die PP2C Nukleinsäuresequenzen der m-RNAs der verschiedenen Subtypen sind im Internet in den einschlägigen Datenbanken (beispielsweise NCBI) verfügbar. Anhand dieser Sequenzen ist es möglich, ein Antisensemolekül nach bekannten Methoden zu designen und ein entsprechendes, mit der m-RNA hybridisierendes Molekül zu erzeugen. Beispiele sind in Fig. 1 aufgeführt.

Vorteilhafterweise kann das die mRNA einer PP2C komplexierende Biomolekül zur Verhinderung der Apoptose in Endothelzellen als Therapeutikum in die Endothelzelle eingebracht werden. Vorzugsweise wird zur Herstellung eines solchen Therapeutikums als Biomolekül ein Oligonukleotid gemäß obigen Ausführungen eingesetzt.

Mit der Erfindung wird femer ein in Zellen exprimierbares Nukleinsäurekonstrukt zur Verfügung gestellt, dessen Expressionsprodukt die mRNA einer PP2C komplexieren kann. Vorzugsweise ist dieses Expressionsprodukt ein einzelsträngiges Oligonukleotid. Dieses kann beispielsweise die Sequenz (vollständig oder partiell) des Gegenstranges einer PP2C m-RNA aufweisen, so daß dieses mit der m-RNA hybridisiert und eine Doppelhelix ausbildet. Beispielsweise seien die Oligonukleotide gemäß Fig. 1 genannt.

Gemäß einer vorteilhaften Weiterbildung ist das erfindungsgemäße Nukleinsäurekonstrukt ein in Eukaryonten exprimierbarer Expressionsvektor. Besonders bevorzugt ist es, wenn die kodierende Sequenz (zumindest partiell) der PP2C in inverser Orientierung hinter den Promoter eines in Eukaryonten exprimierbaren Expressionsvektor einkloniert ist. Dadurch wird direkt beim Ablesen der Sequenz ein Transkript gebildet, welches als Antisensemolekül die mRNA der PP2C komplexieren und somit deren Translation verhindern kann.

Ein solches Nukleinsäurekonstrukt kann zur Herstellung eines Therapeutikums verwendet werden, daß in einem Verfahren zur Verhinderung der Apoptose in Endothelzellen eingesetzt werden kann, wobei eine Endothelzelle mit einem solchen Nukleinsäurekonstrukt transfiziert wird.

In einer weiteren Ausführungsform der vorliegenden Erfindung kann die Phosphorylierung des BAD zu Phospho-BAD gefördert werden, so daß damit das Niveau an pro-aptotischem BAD gezielt auf einem vorteilhaft geringem Wert gehalten werden kann. Dazu wird vorgeschlagen, mittelbar oder unmittelbar auf die Aktivität oder Expression der für die Phosphorylierung des BAD relevanten Proteinkinasen (siehe oben) einzuwirken. Dies kann beispielsweise durch die Induktion oder Steigerung der Enzymexpression oder durch eine Aktivitätssteigerung dieser Enzyme z.B. durch Liganden erfolgen.

In einer Ausführungsform wird zur Herstellung eines Therapeutikums zur Therapie der Atherosklerose das zu den ß-2-Sympathomimetika gehörende Clenbuterol verwendet. In den Endothelzellen kommen β1- und β2-Rezeptoren vor, so daß Clenbuterol in diesen Zellen die Synthese von TGF-β1 induzieren kann. Da bekannt ist, daß Wachstumsfaktoren zu einer Phosphorylierung des BAD führen, kann somit durch die Applikation des Clenbuterols die pro-aptotische Wirkung eines hohen BAD-Niveaus reduziert werden.

Zudem initiiert Clenbuterol - ähnlich wie Adrenalin oder Noradrenalin - als β-Sympathomimetika einen Anstieg des zellulären cAMP-Spiegels, das als Aktivator der Proteinkinase A deren Aktivität steigert. Somit führt Clenbuterol ebenso über diesen Mechanismus zur Erniedrigung des BAD Levels bei gleichzeitiger Erhöhung des Phospho-BAD Werts.

Dieses vorteilhafte Verwendung gilt auch dem Grunde nach für andere Wirkstoffe, die zur einer Erhöhung des zellulären cAMP-Spiegels führen. Dazu gehören beispielsweise Phosphodiesteraseinhibitoren und weitere ß-Sympathomimetika, die erfindungsgemäß eingesetzt werden können, um den Grad an phosphoryliertem BAD in vorteilhafter Weise zu erhöhen. Beispiele für PDE-Inhibitoren sind u.a. Coffein, Theophyllin und Sildenfil. Weitere dieser Wirkstoffe sind aus der einschlägigen Fachliteratur bekannt.

Weitere Substanzen, die erfindungsgemäß zur Erhöhung des Phospho-BAD Werts führen können, sind die HDL Konzentration im Blut erhöhende Substanzen sowie bioaktive Sphingolipide, wie beispielsweise Sylophosphorylcholin und Lysosulfatid. Eine erhöhte HDL-Konzentration und bioaktive Sphingolipide haben durch Steigerung der Kinasenaktivitäten eine Erhöhung des Phospho-BAD Wertes zur Folge.

Sämtliche der genannten Substanzen, Moleküle und Nukleinsäurekonstrukte können daher in vorteilhafter Weise zur Herstellung von Therapeutika zur Behandlung der Atherosklerose eingesetzt werden. Erfindungsgemäß werden femer Therapeutika bzw. pharmakologisch wirksame Substanzen zur Verfügung gestellt, welche wenigstens eine der zuvor genannten Substanzen zur Behandlung der Atherosklerose aufweisen.

Zur Herstellung eines solchen Therapeutikums kann die wirksame Substanz ferner mit zur Herstellung von Arzneimitteln üblichen Trägern kombiniert werden. So kann die Substanz für die jeweils passenden Applikationsarten wie beispielsweise epikutan, bukkal, lingual, sublingual, oral, rektal, pulmonal, konjunktival, per inhalationem, intrakardial, intraarteriell, intravenös, intralumbal, intrahektal, intrakutan, subkutan, intramuskulär oder intraperitoneal aufbereitet werden. Dazu können beispielsweise Lösungen, Suspensionen, Emulsionen, Schäume, Salben, Pasten, Tabletten, Pillen, Dragees, Suspensionen, Suppositorien, Rektalkapseln, Gele, Sprays, Aerosole, Inhalate, Tropfen, Injektionslösung, Infusionslösung, Implantate oder liposomale Systeme hergestellt werden. Für die erfindungsgemäßen Oligotherapeutika sind Formulierungen wichtig, mittels welcher die Nukleinsäuren in die Zellen transportiert werden können. Dies kann beispielsweise mittels Liposomen oder viralen oder nicht viralen Vehikelsystemen erfolgen.

Zur Herstellung eines solchen Therapeutikums kann die wirksame Substanz ferner mit weiteren therapeutisch wirksamen Stoffen kombiniert werden. Als Kombinationspräparat kann die die Dephosphorylierung von Phospho-BAD durch PP2C verhindernde Substanz mit einer erfindungsgemäß die Phosphorylierung des BAD erhöhenden Substanz kombiniert werden. Des weiteren ist es möglich, diese Substanzen alleine oder gemeinsam mit einem bekannten Lipidsenker oder einer Substanz, welche zur Erhöhung der HDL-Konzentration führt, zu kombinieren. Durch ein solches Kombinationspräparat kann zum einen ein Faktor der Atherosklerosebildung (der erhöhte Fettsäurespiegel) und zum anderen den Folgen des hohen Fettspiegels (Apoptose der Endothelzellen) gemeinsam entgegengewirkt werden.

Erfindungsgemäß werden unter *in vitro* Methoden insbesondere Techniken verstanden, welche den Einsatz von Zellen, Zellkulturen, Organen- sowie deren Teile, Organsystemen und Teilen des menschlichen oder tierischen Körpers beinhalten. Der Begriff *in vitro* beinhaltet insbesondere künstliche Systeme unter Verwendung von Zellen, Zellextrakten, gereinigten Komponenten, Homogenaten u.ä..

Der Begriff *Phospho-BAD,* wie er im Zusammenhang mit den erfindungsgemäßen Verfahren verwendet wird, beinhaltet auch den Einsatz etwaiger Phospho-BAD Analoge und weiterer Substrate (bspw. Casein), die von PP2C dephosphoryliert werden.

Die erfindungsgemäß einsetzbaren Substanzen können, sofern chiral, als Racemate und/oder Isomere eingesetzt werden.

Die Erfindung soll nunmehr anhand einiger Ausführungsbeispiele detaillierter erläutert werden:

### Beispiel 1:

Gemäß einer Ausführungsform der erfindungsgemäßen Test-Verfahren erfolgt die Dephosphorylierung von Phospho-BAD durch PP2C *in vitro* unter Verwendung von rekombinantem BAD (Lizcano, J.M., Morrice, N. und Cohen, P. (2000) Regulation of BAD by cAMP-dependant protein kinase is mediated via phosphorylation of a novel site , Ser¹⁵⁵. Biochem.J.Vol:349, 547-557). Phospho-BAD wurde mittels eines Reaktionsansatzes (12µl) erhalten, welcher 12µg BAD (als GST-Fusionsprotein; Lizcano et al., 2000), 0,55µg PKA (Sigma), 1µM ATP, 15µCi [γ-³²P] ATP, 5mM MgCl₂ und 25mM Tris/HCL pH 7,5 enthielt. Der Reaktionsansatz wurde 30min bei 37° inkubiert.

Bei einer alternativen Herstellungsmethode für Phospho-BAD werden 3,4g GST-BAD in 8mM MOPS pH 7.2, 10mM ß-Glycerophosphate, 2mM EDTA, 0,4mM Natriumorthovandat, 15mM MgCl₂, 0,04% 2-Mercaptoethanol, 1µM ATP plus 15µCi [γ-³²P] ATP (zwecks autoradiographischer Detektion) zusammen mit den entsprechenden Kinasen (100ng PKA, 100ng PKB oder 5mU RSK) in einem Gesamtvolumen von 10µl bei 30°C für 30min inkubiert.

Die Phosphorylierungsreaktionen wurden durch Zugabe von 5µl Laemmli Probenpuffer abgestoppt. Radioaktiv markiertes und unmarkiertes ATP wurde im Anschluß an die Inkubation mittels Centri-SEP Zentrifugationssäulchen (von emp Biotech, Berlin) durch Zentrifugation (750 X gₘₐₓ für 2min bei RT) entfernt, um eine erneute Phosphorylierung beim zweiten Inkubationsschritt (mit PP2C) zu vermeiden. Die Proben sollten direkt weiterverarbeitet werden.

Zur Erhaltung des PP2C-Enzyms wurde eine cDNA in *E.coli* exprimiert und mittels Ni-NTA-Sepharose aufgereinigt. Vorliegend wurde die cDNA von PP2Cß aus Rindergehirnen verwendet (J. Neurosci. Res. 51, 328-338 (1998) Protein phosphatase type 2-C isozymes present in vertebrate retinae: purification, characterization and localization in photoreceptors).

Die Dephosphorylierung von Phospho-BAD durch eine PP2C ist sowohl zeitals auch konzentrationsabhängig. Aufgrund der Abhängigkeit der PP2C-Enzymaktivität von der Anwesenheit von Mg²⁺ oder Mn²⁺ lonen, müssen die Testansätze diese enthalten. Vorzugsweise enthält der Testansatz 0,5 bis 1,5mM/l Mg²⁺. Die Inkubationszeit kann wenigstens 10min betragen, beträgt jedoch vorzugsweise 30min. PP2C wurde in einer Konzentration von wenigstens 30ng, vorzugsweise jedoch über 300ng hinzugegeben.

Das radioaktiv markierte abzentrifugierte [³²P]BAD wurde 10-fach mit 25mM Tris/HCL pH 7,5 verdünnt. 15µl Inkubationsansätze wurden für 30min bei 37°C (oder 30°C) inkubiert. Die Testansätze enthielten:
9µl [³²P]BAD (0,2µg)
1,5µl 10-fach Reaktionspuffer ( 200mM Tris/HCL pH 7,5, 100mM MgCl₂,
10% Glycerol, 1% 2-Mercaptoethanol)
300ng PP2C

Die Reaktion wurde durch Zugabe von 5µl Denaturierungspuffer gestoppt und auf Eis gehalten, bis die Proteine auf einem 12,5% SDS PAGE Minigel separiert wurden. Der Grad der Dephosphorylierung bzw. Phosphorylierung wurde mittels Autoradiographie untersucht.

Gemäß einem weiteren möglichen *in vitro* Testansatz wird die Aktivität von PP2C durch Inkubation eines 30µl Ansatzes enthaltend 20mM Tris-HCL pH 7.5, 0,01% 2-Mercaptoethanol, 1,3mg/ml BSA und 1µM [³²P] Casein (5 X 10⁴cpm) bei 30°C für 10min untersucht (McGowan and Cohen, 1988; Cohen 1991). Zur Bestimmung der PP2C Aktivität in Verbindung mit Fettsäuren wurde 0,7mM Magnesiumacetat zugegeben. Die Reaktionen wurden durch Zugabe von 200µl 20% Trichloressigsäure abgestoppt. Nach Zentrifugation bei 10000 g (5min) wurden 200µl des Überstandes auf seinen [³²P] Phosphatgehalt untersucht.

Anhand des jeweiligen Ergebnisses kann überprüft werden, ob das Testsystem funktionsfähig ist. Die auf ihre die Dephosphorylierungsreaktion inhibierende Wirkung zu testenden Substanzen werden einem entsprechenden Testansatz zugegeben und wie oben beschrieben inkubiert. Mittels Autoradiographie wird im Anschluß überprüft, inwieweit eine Inhibition im Vergleich zu den Kontrollen (ohne Testsubstanz) stattgefunden hat. Vorzugsweise wird die potentielle Wirksubstanz in verschiedenen Konzentrationen getestet, um zu vermeiden, daß eine guter Kandidat deshalb "übersehen" wird, weil er in der falschen Konzentration keinen Effekt hat. Kandidaten, insbesondere erfolgversprechende, können auch auf ihre synergistische Wirkung gemeinsam in einem Testansatz getestet werden.

Um zu testen, inwieweit eine gezielte Inhibition der durch Fettsäuren aktivierten PP2C möglich ist, wurde dem Testansatz femer eine die PP2C aktivierende Fettsäure zugegeben. So beispielsweise Ölsäure, vorzugsweise in einer Konzentration von 10 bis 500µM. Durch entsprechende Kontrollen kann bestimmt werden, inwieweit die Testsubstanz nur gezielt die Fettsäureaktivierung unterbindet, jedoch die "normale" Aktivität von PP2C wenig oder gar nicht inhibiert. Durch einen solchen Testansatz können Substanzen ermittelt werden, welche gezielt nur die durch Fettsäure induzierte Apoptose inhibieren, jedoch normale regulatorische Prozesse nicht beeinflussen.

Die so identifizierten Testsubstanzen können im Anschluß an Endothelzellen getestet werden (siehe Bsp. 2).

### Beispiel 2:

Endothelzellkulturen wurden suspensiert und die Zellkultur wurde in einer Dichte von 4 X 10⁴ Zellen/cm² auf Poly-L-Lysin beschichteten Kolben oder Poly-L-Lysin beschichteten Deckgläschen ausgesät, welche für TUNEL Assays in Petrischalen plaziert wurden. Die Kulturen wurden bei 37°C und 5% CO₂ in einer befeuchteten Atmosphäre in DMEM gehalten, wobei 20% fötales Serum und Antibiotika zugegeben wurden. Das Medium wurde alle 2 Tage ausgewechselt.

4 Tage nach der Aussaat wurden die Kulturen einem serumfreien Medium für 24h ausgesetzt. Im Anschluß wurden die Zellen mit den Testsubstanzen und/oder PP2C aktivierenden Fettsäuren, Fettsäurederivaten oder Bindemitteln behandelt. Fettsäuren und/oder deren Derivate wurden zunächst in reinem DMSO aufgelöst und dann in Kulturmedium verdünnt, so daß die Endkonzentration bei 0,016% DMSO in allen Experimenten lag.

Zur Induktion der Apoptose werden vorzugsweise die PP2C aktivierende Fettsäuren (wie beispielsweise Öl-oder Ginkolsäure) zugegeben, jedoch sind auch andere bekanntermaßen die Apoptose induzierenden Wirkstoffe als Induktor einsetzbar. Zum Nachweis von PP2C in Endothelzellkulturen wurden 20mM Mg²⁺ Ionen eingesetzt. Im Anschluß an die Inkubation wurde untersucht, inwieweit die Testsubstanzen die Apoptose inhibieren können.

Die Kernform und die Chromosomenstruktur kann durch Färbung der nuklären DNA mit Hoechst 33258 visualisiert werden. Die Kulturen wurden für 10min mit 10µg/ml Hoechst 33258 in Methanol inkubiert und dann mit Methanol und PBS gewaschen. Im Anschluß wurde die nukläre Morphologie unter einem Fluoreszenzmikroskop untersucht. Die Anzahl der Endothelzellen mit geschrumpften Nuclei und kondensierten Chromatin wurden in drei Bereichen in jedem von vier verschiedenen Kulturkolben gezählt. Die Resultate wurden als das Prozentverhältnis von Endothelzellen mit kondensierten Chromatin und geschrumpften Kern gegenüber der Gesamtanzahl der Zellen ausgedrückt und ausgewertet.

Zum Nachweis der DNA Fragmentierung wurden TUNEL Assays durchgeführt, wobei ein kommerziell erhältlicher Kit nach Angaben des Herstellers verwendet wurde. Zellmonolayers wurden in Methanol bei -20°C für 20min kurz fixiert und dann bei 37°C für 1 h mit Digoxigenin markierten ddUTP in Anwesenheit einer terminalen Transferase inkubiert. Die Reaktion wurde abgestoppt und Anti-Digoxigenin-Antikörper, gekoppelt mit Fluorescin Isothiocyanat, wurden zugegeben und für weitere 30min inkubiert. Im Anschluß wurde die Fluoreszenz mit einem geeignetem Mikroskop untersucht (LSM 510, Zeiss, Deutschland).

Kaspase-3 Aktivität wurde mittels einem fluorimetrischem Versuchskit bestimmt. Der Assay basierte auf der Hydrolyse des Peptidsubstrates Acetyl-Asp-Glu-Val-Asp-7-Amido-4-Methylcoumarin (Ac-DEVD-AMC) durch Caspase-3, wodurch die fluoreszierende AMC Komponente freigesetzt wird. Die

Zellen wurden geerntet und durch Zentrifugation bei 600 X g für 5min bei 4°C pelletiert. Der Überstand wurde abgenommen und das Pellet mit PBS gewaschen. Im Anschluß wurde das Pellet in einem Lysispuffer gelöst, welcher 50mM HEPES, pH 7.4, 5mM 3-(3 Cholamidopropyl) Dimethylammonio-1-Propansulfonat und 5mM Dithiothreitol enthielt und für 20min auf Eis inkubiert. Im Anschluß wurden die Zellen bei 14000 X g für 15min bei 4°C zentrifugiert. Der Überstand wurde mit dem Reaktionspuffer (10mM Ac-DEVD-AMC, 20mM HEPES, pH 7.5, 2mM EDTA, 0,1% 3-(3-Cholamidopropyl)Dimethylammonio-1-Propansulfonat und 5mM Dithiothreitol) gemischt. Die Fluoreszenz des AMC wurde bei einer Anregungswellenlänge von 360nm und einer Emmissionswellenlänge von 460nm gemessen. Die Resultate wurden mittels einer AMC Standardkurve berechnet und werden vorzugsweise als nmol AMC/mg protein/min. Die Proteinmenge wurde mittels des "bicincolinic acid protein assay" Kit gemessen, wobei Rinderserumalbumin als Standard diente.

Mittels der beschriebenen Methoden kann bestimmt werden, ob bei den behandelten Zellen apoptotische Prozesse ablaufen. Dadurch können Testsubstanzen aufgefunden werden, welche die Apoptose in Endothelzellen verringern oder verhindern. Dadurch können wertvolle potentielle Therapeutika der Atherosklerose identifiziert werden.

## Patentansprüche

1. Verwendung einer Substanz, die die Dephosphorylierung von Phospho-BAD in Endothelzellen verhindert oder verringert, zur Herstellung eines Therapeutikums zur Verhinderung der Atherosklerose oder Verringerung der Atherosklerosegefahr.

2. Verwendung einer Substanz, die die Phosphorylierung von BAD in Endothelzellen verstärkt, Zur Herstellung eines Therapeutikums zur Verhinderung der Atherosklerose oder Verringerung der Atherosklerosegefahr.

3. Verwendung nach einem der vorhergehenden Ansprüche **dadurch gekennzeichnet, daß** die Substanz ein Antisense Oligonukleotid ist.

4. Verwendung nach Anspruch 3, **dadurch gekennzeichnet, daß** das Antisense Oligonukleotid mit der mRNA einer PP2C hybridisiert.

5. Verwendung nach einem der Ansprüche 1 oder 2, **dadurch gekennzeichnet, daß** die Substanz die Phosphorylierung/Dephosphorylierung von Ser¹¹², Ser¹³⁶ und/oder Ser¹⁵⁵ des BAD verändert, so daß die phosphorylierte Form des BAD begünstigt wird.

6. Verwendung nach einem der Ansprüche 1, 2 oder 5 **dadurch gekennzeichnet, daß** die Substanz ein Serin-Threonin Phosphatase-Inhibitor ist.

7. Verwendung nach Anspruch 6, **dadurch gekennzeichnet, daß** die Substanz ein Inhibitor der PP2C ist.

8. Verwendung nach Anspruch 2, **dadurch gekennzeichnet, daß** die Substanz ausgewählt ist aus der Gruppe der ß-Sympatomimetika, ß-2-Sympathomimetika, insbesondere Clenbuterol und Phosphodiesterasehemmer.

9. Verfahren zur Identifizierung einer Substanz, welche die Dephosphorylierung von Phospho-BAD durch PP2C inhibiert, **gekennzeichnet durch**
a. Inkubieren von PP2C mit Phospho-BAD oder einem umsetzbaren Analogon und wenigstens einer Testsubstanz in vitro,
b. Nachweisen der Wirksamkeit der Testsubstanz in Bezug auf die Dephosphorylierung des Phospho-BAD **durch** eine PP2C.

10. Verfahren zur Identifizierung einer Substanz, welche die apoptotischen Vorgänge in Endothelzellen durch Hemmung der Dephosphorylierung von Phospho-BAD durch PP2C beeinflussen kann, **gekennzeichnet durch**
a. Induzieren der Apoptose in Endothelzellen,
b. in Kontakt bringen mit einer oder mehreren Testsubstanzen und inkubieren,
c. Nachweisen der Wirksamkeit der Testsubstanz in Bezug auf die Dephosphorylierung des Phospho-BAD oder eines Analogons **durch** eine PP2C.

11. Verfahren nach Anspruch 10, **dadurch gekennzeichnet, daß** nach Schritt a.) ein enzymatisch aktiver Zellextrakt aus den induzierten Endothelzellen gewonnen wird.

12. Verfahren nach einem oder mehreren der vorherigen Ansprüche, **dadurch gekennzeichnet, daß** zur Induktion in Schritt a.) wenigstens eine die PP2C aktivierende Fettsäure eingesetzt wird, vorzugsweise eine solche, die eine Länge von wenigstens 15 C-Atomen aufweist, mindestens einfach ungesättigt ist und wenigstens eine freie Carboxylgruppe aufweist, vorzugsweise Ölsäure, Arachidonsäure und/oder Ginkolsäure.

13. Verfahren zur Identifizierung einer Substanz, welche die Dephosphorylierung von Phospho-BAD durch eine PP2C inhibiert, **gekennzeichnet durch**
a. Screenen einer Substanzbibliothek mit einer PP2C oder einem PP2C-Fragment als Target zur Identifizierung von Bindungspartnern,
b. Inkubatieren der in Schritt a) isolierten Substanzen mit PP2C und Phospho-BAD oder einem umsetzbaren Analogon,
c. Nachweisen der Wirksamkeit der Dephosphorylierung des Phospho-BAD oder des umsetzbaren Analogons **durch** die in Schritt a) isolierte Substanz.

14. Verfahren zur Identifizierung einer Substanz, welche die Dephosphorylierung von Phospho-BAD durch eine PP2C inhibiert, **gekennzeichnet durch**
a. Screenen einer Substanzbibliothek mit Phospho-BAD oder einem phosphorylierten Peptidfragment von BAD als Target zur Identifizierung von Bindungspartnern, die mit Phospho-BAD interagieren,
b. Inkubieren der in Schritt a) isolierten Substanz mit PP2C und Phospho-BAD oder einem umsetzbaren Analogon,
c. Nachweisen der Wirksamkeit in Bezug auf die Dephosphorylierung des Phospho-BAD oder des umsetzbaren Analogons **durch** PP2C.

15. Verfahren zur Herstellung eines Therapeutikums zur Behandlung der Atherosklerose, **gekennzeichnet durch**
a. Identifizieren einer Substanz, welche die apoptotischen Vorgänge in Endothelzellen **durch** Hemmung der Dephosphorylierung von Phospho-BAD **durch** PP2C reguliert, **durch** ein Verfahren nach wenigstens einem der Ansprüche 9 bis 14,
b. Vermischen dieser Substanz mit wenigstens einer physiologisch verträglichen Trägersubstanz zu einem Arzneimittel.

16. Verfahren nach einem oder mehreren der obigen Ansprüche, **dadurch gekennzeichnet, daß** PP2C aus der Gruppe von PP2Cα; PP2Cβ; PP2C γ; PP2Cδ sowie deren Subtypen ausgewählt wird.

17. Verfahren nach einem oder mehreren der obigen Ansprüche, **dadurch gekennzeichnet, daß** Phospho-BAD oder Phospho-BAD Peptidfragmente eingesetzt werden, welche in der Position Serin¹⁵⁵, Serin¹¹² und/oder Serin¹³⁶ phosphoryliert sind.

18. Verfahren nach Anspruch 17, **dadurch gekennzeichnet, daß** zum Screenen Peptidfragmente des BAD aus dem Aminosäurebereich um Ser¹¹², Ser¹³⁶, Ser¹⁵⁵ eingesetzt werden.

19. Verwendung einer mittels eines Verfahrens nach einem oder mehreren der obigen Ansprüche generierbaren Substanz, welche die Dephosphorylierung von Phospho-BAD durch eine PP2C verhindert, zur Herstellung eines Therapeutikums zur Behandlung der Atherosklerose.

20. Verwendung einer Substanz zur Herstellung eines Therapeutikums zur Inhibierung der Apoptose in Endothelzellen, **dadurch gekennzeichnet, daß** sie ein Inhibitor einer PP2C ist.

21. Verwendung nach Anspruch 20, **dadurch gekennzeichnet, daß** die Substanz ein Inhibitor einer durch Fettsäuren aktivierbaren PP2C ist.

22. Verwendung nach Anspruch 20 oder 21, **dadurch gekennzeichnet, daß** die Substanz kompetitiv die Bindung von Fettsäuren an PP2C verhindert.

23. Verwendung nach einem oder mehreren der Ansprüche 20 bis 22, **dadurch gekennzeichnet, daß** die Substanz eine Fettsäure, eine modifizierte Fettsäure, ein Fettsäurederivat, ein Biphosphonatfettsäurederivat oder ein Fettsäureanalog ist.

24. Verwendung nach Anspruch 23, **dadurch gekennzeichnet, daß** die Substanz eine Biphosphonsäuren oder ein Derivat einer solchen Säure gemäß der allgemeinen Formel 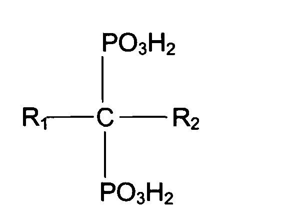 ist, in welcher
R1
- ein linearer oder verzweigter Alkylrest mit 1 bis 12 Kohlenstoffatomen ist, der gegebenenfalls substituiert sein kann durch Substituenten wie Aminogruppen, N-Mono- bzw. Dialkylaminogruppen, wobei die Alkylgruppen 1 bis 12 C-Atome und/oder SH-Gruppen enthalten können, oder
- ein substituierter oder unsubstituierter carbo- oder heterocyclischer Arylrest, der ggf. ein oder mehrere Heteroatome und als Substituenten verzweigte und/oder unverzweigte Alkylreste mit 1 bis 12 C-Atomen, freie oder mono- respektive dialkylierte Aminogruppen mit 1 bis 6 C-Atomen oder Halogenatome aufweisen kann und
R₂
- gleich OH, ein Halogenatom, vorzugsweise Cl, H oder NH₂ ist.

25. Verwendung nach Anspruch 23, **dadurch gekennzeichnet, daß** die Substanz ausgewählt ist aus der Gruppe aus Ibandronsäure, Etidronsäure, Clodronsäure, Pamidronsäure, Alendronsäure, Risedonsäure, Tiludronsäure, Zoledronsäure, Cimadronsäure, Neridronsäure, Olpadronsäure, 3-Pyrrol-1-hydroxypropan-1-1-diphosphonsäure und/oder Minodronsäure, Diphosphonat-1,1-Dicarbonsäure (DPD) und/oder 1-Methyl-1-hydroxy-1.1-diphosphonsäure (MDP).

26. Verwendung nach einem oder mehreren der vorherigen Ansprüche, **dadurch gekennzeichnet, daß** die Substanz ein negativer Regulator einer durch Fettsäuren aktivierbaren PP2C ist.

27. Verwendung nach einem oder mehreren der vorherigen Ansprüche, **dadurch gekennzeichnet, daß** die Substanz an das aktive Zentrum der PP2C bindet und/oder kompetitiv die Bindung von Phospho-BAD an PP2C unterbindet.

28. Verwendung nach Anspruch 27, **dadurch gekennzeichnet, daß** sie in ihrer dreidimensionalen Struktur im wesentlichen der dreidimensionalen Struktur des Phospho-BAD entspricht und ein nicht oder nur langsam umsetzbares Substratanalog ist.

29. Verwendung einer Substanz zur Herstellung eines Therapeutikums zur Inhibierung der Apoptose in Endothelzellen, **dadurch gekennzeichnet, daß** sie an Phospho-BAD bindet und die Phosphatgruppe, insbesondere bei P-Ser¹⁵⁵, für eine PP2C unzugänglich macht.

30. Verwendung nach Anspruch 29, **dadurch gekennzeichnet, daß** die Substanz ein Polypeptid ist.

31. Verwendung nach Anspruch 29 oder 30, **dadurch gekennzeichnet, daß** die Substanz mittels eines Interaktionsbereichs, vorzugsweise einer Bindungstasche, an Phospho-BAD bindet.

32. Verwendung nach Anspruch 31, **dadurch gekennzeichnet, daß** die Bindungstasche in ihrer dreidimensionalen Struktur im wesentlichen der Antigenbindungsstelle von Antikörpern gegen Phospho-BAD entspricht.

33. Verwendung nach Anspruch 31 oder 32, **dadurch gekennzeichnet, daß** die Bindungstasche in ihrer dreidimensionalen Struktur im wesentlichen der Antigenbindungsstelle eines Antikörpers gegen Phospho-BAD Serin¹¹², Phospho-BAD Serin¹³⁶ und/oder Phospho-BAD Serin¹⁵⁵ entspricht.

34. Verwendung einer Substanz zur Herstellung eines Therapeutikums zur Inhibierung der Apoptose in Endothelzellen, **dadurch gekennzeichnet, daß** die Substanz ein an PP2C bindendes Polypeptid ist.

35. Verwendung nach Anspruch 34, **dadurch gekennzeichnet, daß** die Substanz ein Antikörper oder Antikörperfragment ist.

36. Verwendung einer Substanz zur Herstellung eines Therapeutikums zur Inhibierung der Apoptose in Endothelzellen, **dadurch gekennzeichnet, daß** es ein Biomolekül ist, daß die mRNA einer PP2C komplexiert.

37. Verwendung einer Substanz nach einem oder mehreren der Ansprüche 20 bis 36, **dadurch gekennzeichnet, daß** die Substanz gemäß einem Verfahren nach einem oder mehreren der Ansprüche 9 bis 15 herstellbar ist.

38. Verwendung nach Anspruch 36, **dadurch gekennzeichnet, daß** das Biomolekül eine zumindest teilweise einzelsträngige Nukleinsäure ist, die eine Nukleinsäuresequenz aufweist, die vollständig oder partiell dem Gegenstrang einer PP2C m-RNA entspricht, so daß dieses die PP2C m-RNA hybridisiert und mit dieser zumindest bereichsweise eine Doppelhelix ausbildet.

39. Verwendung eines in Zellen exprimierbaren Nukleinsäurekonstrukts zur Herstellung eines Therapeutikums zur Behandlung der Atherosklerose, **dadurch gekennzeichnet, daß** das Expressionsprodukt ein Biomolekül ist, welches die PP2C mRNA komplexiert.

40. Verwendung nach Anspruch 39, **dadurch gekennzeichnet, daß** das Expressionsprodukt des Nukleinsäurekonstrukts ein einzelsträngiges Oligonukleotid ist.

41. Verwendung nach Anspruch 39 oder 40, **dadurch gekennzeichnet, daß** das Expressionsprodukt des Nukleinsäurekonstrukts eine Nukleinsäure mit einer Nukleinsäuresequenz gemäß einer den in Fig. 1 aufgeführten Sequenzen ist.

42. Verwendung wenigstens einer Substanz oder eines Nukleinsäurekonstrukts nach wenigstens einem der vorherigen Ansprüche zur Herstellung eines Therapeutikums zur Behandlung der Atherosklerose und/oder der damit in Zusammenhang stehenden Krankheiten.

43. Pharmakologisch wirksame Zusammensetzung zur Behandlung der Atherosklerose, aufweisend wenigstens eine Substanz oder eines Nukleinsäurekonstruktes nach wenigstens einem der vorherigen Ansprüche.

44. Zusammensetzung nach Anspruch 43, **dadurch gekennzeichnet, daß** sie wenigstens einen Lipidsenker und/oder einen die HDL-Konzentration erhöhenden Wirkstoff aufweist.

45. Verwendung von ß-Sympathomimetika, ß-2-Sympathomimetika, insbesondere Clenbuterol und/oder Phosphodiesterasehemmer zur Herstellung eines Therapeutikums, daß den Gehalt an Phospho-BAD in einer Endothelzelle erhöht, zur Verhinderung der Apoptose in Endothelzellen.
